# EUROPEAN PATENT SPECIFICATION

(11) **EP 0 705 345 B1**
(45) Date of publication and mention of the grant of the patent: **14.04.1999**
(21) Application number: 94919409.6
(22) Date of filing: 15.06.1994
(51) Int. Cl.: C12P 7/40

(54) **PROCESS FOR THE PREPARATION OF PYRUVIC ACID**
VERFAHREN ZUR HERSTELLUNG VON BRENZTRAUBENSÄURE
PROCEDE DE PREPARATION DE L'ACIDE PYRUVIQUE

(30) Priority: 25.06.1993 US 82879
(43) Date of publication of application: 10.04.1996
(73) Proprietor: E.I. DU PONT DE NEMOURS AND COMPANY, Wilmington Delaware 19898 (US)
(72) Inventor: ANTON, David, Leroy, Wilmington, DE 19803 (US); DiCOSIMO, Robert, Wilmington, DE 19808 (US); WITTERHOLT, Vincent, Gerard, Wilmington, DE 19803 (US)
(74) Representative: Woodman, Derek
(86) International application number: US9406436
(87) International publication number: WO9500656

(56) References cited:
- EP-A- 0 342 984
- US-A- 4 900 668
- CHEMICAL ABSTRACTS, vol. 47, no. 2, 10 September 1953, Columbus, Ohio, US; abstract no. 8805h, I. ZELITCH ET AL. 'Oxidation and reduction of glycolic and glyoxylic acids in plants. I. Glycolic acid oxidase.' & JOURNAL OF BIOLOGICAL CHEMISTRY., vol.201, 1953, BALTIMORE US pages 707 - 718 cited in the application
- CHEMICAL ABSTRACTS, vol. 43, no. 16, 25 August 1949, Columbus, Ohio, US; abstract no. 6267a, C.CLAGETT ET AL. 'Oxidation of alpha-hydroxy acids by enzymes from plants.' & JOURNAL OF BIOLOGICAL CHEMISTRY., vol.178, 1949, BALTIMORE US pages 977 - 987 cited in the application
- CHEMICAL ABSTRACTS, vol. 44, no. 7, 10 April 1950, Columbus, Ohio, US; abstract no. 3050f, N. TOLBERT ET AL. 'Products of the oxidation of glycolic acid and l-lactic acid by enzymes from tobacco leaves.' & JOURNAL OF BIOLOGICAL CHEMISTRY., vol.181, 1949, BALTIMORE US pages 905 - 914 cited in the application
- BIOTECH. LETT., vol.9, no.4, 1987, page 253 - 258, B. A. BURDICK AND J. R. SCHAEFFER 'CO-IMMOBILIZED COUPLED ENZYME SYSTEMS ON NYLON MESH CAPABLE OF GLUCONIC AND PYRUVIC ACID PRODUCTION.'
- J. BIOL. CHEM., vol.236, no.4-6, 1961, page 1280 - 1284, K. E. RICHARDSON AND N. E. TOLBERT 'OXIDATION OF GLYOXYLIC ACID TO OXALIC ACID BY GLYOXYLIC ACID OXIDASE.'
- J. BIOL. CHEM., vol.181, no., 1949, page 905 - 914, N. E. TOLBERT ET AL. 'PRODUCTS OF THE OXIDATION OF GLYCOLIC ACID AND L-LACTIC ACID BY ENZYMES FROM TOBACCO LEAVES.'
- J. BIOL. CHEM., vol.178, no., 1949, page 977 - 987, C. O. CLAGETT ET AL. 'OXIDATION OF ALPHA-HYDROXY ACIDS BY ENZYMES FROM PLANTS.'

## Description

### BACKGROUND OF THE INVENTION

### Field of the Invention:

This invention relates to a process for the production of pyruvic acid, where L-lactic acid and oxygen are reacted in an aqueous solution in the presence of catalysts consisting of glycolate oxidase ((S)-2-hydroxy-acid oxidase, EC 1.1.3.15) and catalase (EC 1.11.1.6).

### Description of the Related Art:

Pyruvic acid has been prepared by the fermentation of various carbon sources (e.g., glucose, yeast extracts and peptone), but these methods usually produce pyruvic acid in low yields (based on added carbon source) and in relatively low concentrations as one component of a mixture of fermentation products. Separation and isolation of pyruvic acid from such complex fermentation broths are generally difficult and expensive to perform.

The preparation of pyruvic acid via the microbiological oxidation of optically pure D(-)-lactic acid has been described by Cooper (U.S. Patent 4,900,668; Feb. 13, 1990). Although this process improves upon other fermentation routes by not utilizing the D-lactic acid as a carbon source to produce the cell mass necessary for the reaction, a growth medium containing a second carbon source (e.g., D(-)-mannitol and corn steep liquor) are required for both the production of cell mass as well as the fermentative conversion of D-lactic acid. Additionally, D-lactic acid is not as ubiquitous in nature, and is much more expensive to produce or purchase, than L(+)-lactic acid.

EP 342,984 discloses an enzymatic assay used to determine the concentration of NAD(P)H in a sample. The assay uses as a reagent a mixture containing lactate oxidase. This is not the oxidase of the present invention.

The conversion of L-lactic acid to pyruvic acid has been demonstrated using the enzyme L-lactate oxidase (L-lactate: oxygen oxidoreductase, non-decarboxylating, EC 1.1.3.2) as catalyst (B. A. Burdick and J. R. Schaeffer Biotech. Lett., Vol. 9, 253-258 (1987)). L-lactate oxidase (from *Pediococcus*) catalyzes the oxidation of L-lactate by oxygen to pyruvate and hydrogen peroxide: 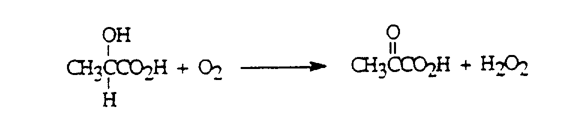 The L-lactate oxidase was co-immobilized with catalase (oxidase:catalase = 1:281 (IU/IU)) to limit oxidation of pyruvate by byproduct hydrogen peroxide, which produces acetate and carbon dioxide. The oxidation of 49 mM solutions of L-lactate in 0.1 M phosphate buffer at pH 7 resulted in up to 47 % yields of pyruvic acid (isolated as the 2,4-dinitrophenylhydrazone derivative).

Glycolate oxidase ((S)-2-hydroxy-acid oxidase, EC 1.1.3.15), an enzyme commonly found in leafy green plants and mammalian cells, catalyzes the oxidation of glycolic acid to glyoxylic acid. This same enzyme also catalyzes the oxidation of L-lactic acid to pyruvic acid, with the concomitant production of hydrogen peroxide. C. O. Clagett, N. E. Tolbert and R. H. Burris, J. Biol. Chem., Vol. 178, 977-987 (1949) first reported an α-hydroxy acid oxidase, extracted from a variety of green leafy plants, which catalyzed the oxidation of glycolic acid, and was also specific for the L-isomer of lactic acid. The pH optimum for the oxidation of 80 mM *dl*-lactate was 7.6; no reaction products were identified or isolated. N. E. Tolbert et al., J. Biol. Chem., Vol. 181, 905-914 (1949) employed a purified α-hydroxy acid oxidase from tobacco leaves for the oxidation of ca. 113 mM *dl*-lactic acid in phosphate buffer at pH 8; an unreported quantity of pyruvic acid was isolated from the reaction as a 2,4-dinitrophenylhydrazone, and a significant amount of carbon dioxide was also produced. K. E. Richardson and N. E. Tolbert, J. Biol. Chem., Vol. 236, 1280-1284 (1961) later reported that this α-hydroxy acid oxidase was more commonly referred to as glycolic acid oxidase (i.e., glycolate oxidase).

I. Zelitch and S. Ochoa, J. Biol. Chem., Vol. 201, 707-718 (1953) reported that glycolic acid oxidase catalyzes the oxidation of L-lactic acid by molecular oxygen to produce pyruvic acid and hydrogen peroxide, and that in the absence of catalase, the peroxide reacts non-enzymatically with pyruvate to form acetate, CO₂, and water. Flavin mononucleotide (FMN) was identified as a required enzyme cofactor, and the addition of FMN to aqueous solutions of the enzyme greatly increased the stability of glycolic acid oxidase. The oxidation of 3.3 mM solutions of L-lactate in 50 mM phosphate buffer (pH 8.0) and in the presence of an excess of added catalase produced 3.2 mM pyruvic acid (determined colorimetrically); no product was isolated.

The oxidation of lactate to pyruvate has also been demonstrated using an L-α-hydroxy acid oxidase isolated from rat kidney (M. Blanchard et. al., J. Biol. Chem., Vol. 163, 137-144 (1946)). The oxidation of a 33 mM solution of lactate in 0.167 M phosphate buffer at pH 8.0 and in the presence of added excess catalase produced pyruvate in 79 % yield (isolated as the 2,4-dinitrophenylhydrazone derivative) Additional references to the oxidation of lactic acid to pyruvic acid catalyzed by soluble enzymes include: J. C. Robinson et al., J. Biol. Chem., Vol. 237, 2001-2010 (1962) (hog renal cortex L-α-hydroxy acid oxidase), P. Urban et. al., Biochemistry, Vol. 27, 7365-7371 (1988) (rat kidney L-α-hydroxy acid oxidase), D. W. Fry and K. E. Richardson, Biochim. Biophys. Acta, Vol. 568, 135-144 (1979) (human liver glycolic acid oxidase), M. J. Emes and K. H. Erismann, Int. J. Biochem., Vol. 16, 1373-1378 (1984) *(Lemna minor* L glycolate oxidase), H. S. Kim and J. D. Choi, Korean Biochem. J., Vol. 20, 350-356 (1987) (spinach glycolate oxidase).

Although the enzyme-catalyzed oxidation of L-lactic acid by oxygen is well-known, a high selectivity to pyruvic acid has only been demonstrated in one experiment (I. Zelitch and S. Ochoa) where the concentration of L-lactate was 3.3 mM; this low concentration of L-lactate limited the concentration of hydrogen peroxide formed, and in the presence of an excess of catalase, also limited the reaction of hydrogen peroxide with pyruvate to produce acetate and carbon dioxide. The recovery of such a low concentration of pyruvate (ca. 3.2 mM) from an aqueous reaction mixture is impractical for an economical manufacturing process.

### Summary Of The Invention

This invention relates to the preparation of pyruvic acid (or a salt thereof, as explained more fully hereafter) by oxidizing L-lactic acid (or a salt thereof) with oxygen in aqueous solution and in the presence of the two enzyme catalysts glycolate oxidase (e.g., (S)-2-hydroxy-acid oxidase, EC 1.1.3.15) and catalase (e.g., EC 1.11.1.6). Thus the present invention provides an improved process for the production of pyruvic acid comprising the steps of reacting, in an aqueous solution, L-lactic acid and oxygen in the presence of the enzyme catalyst glycolate oxidase and the enzyme catalyst catalase for a time sufficient to convert the L-lactic acid to pyruvic acid at high yields, wherein the initial concentration of the L-lactic acid is from about 0.1 to about 2.0 M and then recovering the pyruvic acid. The embodiments of the invention are specified in the claims. It should be appreciated that for purposes of this invention the use of the terms pyruvic acid and L-lactic acid particularly when referring to an aqueous solution at pH range of 6 to 10 more specifically refer to a highly dissociated state wherein a partially neutralized acid solution is predominantly present as the pyruvate ion and the L-lactate ion, respectively. Pyruvic acid is useful as a chemical intermediate in the preparation of fine chemicals, agrochemicals and pharmaceuticals. Therefore it should be further appreciated that for purposes of this invention, the reference to high yields and recovery of pyruvic acid are intended to include as equivalent a process wherein the pyruvic acid is inherently produced as an intermediate to an otherwise useful derivative compound of pyruvic acid at correspondingly high yields.

According to the present invention, pyruvic acid has been prepared via the enzyme-catalyzed oxidation of L-lactic acid in concentrations of up to 0.5 M, and isolated in 96 % yield (98 % purity, sodium salt). The high initial concentration of L-lactic acid employed might have been expected to result in substrate inhibition of the glycolate oxidase, which would have limited the reaction rate and/or the final concentration of product. Similarly, a 0.5 M concentration of pyruvic acid might have resulted in product inhibition of the enzyme, again limiting the concentration of product obtained.

Further according to the present invention, it has been discovered that yields of pyruvate as high as 98-99 % can be obtained in unbuffered reactions run with no pH control; all previous examples of enzymatic oxidations of L-lactate have been performed using a buffer, usually phosphate buffer. The high yields of pyruvate obtained in the absence of buffer equal or exceed those obtained in the presence of added buffer. The preparation of pyruvate in the absence of added buffer allows for a simple isolation of product from a reaction mixture; the catalyst is removed by filtration or centrifugation, leaving an aqueous solution of a pyruvic acid salt that is easily recovered by removal of the water (for example, by stripping of water under reduced pressure, by lyophilization, or the like).

The use of genetically-engineered, permeabilized whole-cell catalysts (i.e., a microbial transformant containing both glycolate oxidase and catalase) for the oxidation of L-lactate to pyruvate has been demonstrated. The use of these whole-cell catalysts has provided a number of advantages over the use of soluble enzymes as catalysts in the present invention. The solutions containing whole-cell catalyst can be sparged with oxygen or an oxygen-containing gas, which increases the reaction rate; sparging a reaction mixture containing soluble enzymes results in the rapid, irreversible denaturation of glycolate oxidase. At the conclusion of the reaction, the whole-cell catalyst is easily recovered for reuse by filtration or centrifugation, while the soluble enzymes cannot be centrifuged out, and filtration results in the loss of much of the soluble glycolate oxidase activity. The recovery of reusable enzyme activities for the whole-cell catalysts is extremely high after each catalyst recycle (ca. 95 % or greater), while the measured activity of remaining soluble glycolate oxidase after one reaction is typically 40 % to 60 %. The use of glycolate oxidase and catalase attached to or immobilized on an inert support will exhibit many of these same advantages and as such are to be considered equivalent for purposes of this invention.

A comparison of the results obtained from the oxidation of 0.50 M L-lactate solutions using 1) soluble glycolate oxidase (g.o.) and soluble catalase, 2) *Hansenula polymorpha* permeabilized-cell catalyst, and 3) *Pichia pastoris* permeabilized-cell catalyst under identical reaction conditions is provided by the following table (see Examples 5, 12, and 13 for reaction conditions):

| catalyst | g.o. (IU/mL) | catalase (IU/mL) | reaction time (h) | pyruvate (%) | acetate (%) | lactate (%) |
|---|---|---|---|---|---|---|
| soluble enzymes | 6.0 | 10,000 | 5 | 95.3 | 4.5 | 0.9 |
| *H. polymorpha* | 6.4 | 5,000 | 2 | 97.0 | 2.5 | 0.4 |
| *Pichia pastoris* | 6.3 | 10,000 | 3 | 98.2 | 1.2 | 0.6 |

The yield of pyruvate is higher, and the production of byproduct acetate is lower, when using either permeabilized catalyst when compared to using soluble glycolate oxidase and catalase as catalyst. When using the *H. polymorpha* permeabilized-cell catalyst at the same glycolate oxidase concentration as for the soluble enzyme experiment, less acetate (produced by the reaction of byproduct hydrogen peroxide with pyruvate) is formed, even though the concentration of endogenous cellular catalase present in the reaction mixture is half of that in the soluble enzyme reaction. These results, and those in the accompanying Examples, demonstrate the unexpected improvement in pyruvate yields when using permeabilized-cell transformants as catalysts.

### Description Of The Preferred Embodiments

The catalytic oxidation of L-lactic acid or a suitable salt thereof is conveniently carried out by contacting the L-lactic acid with a source of molecular oxygen in the presence of an enzyme catalyst which catalyzes the reaction of L-lactic acid with O₂ to form pyruvic acid. One such catalyst is the enzyme glycolate oxidase (EC 1.1.3.15), also known as glycolic acid oxidase. Glycolate oxidase may be isolated from numerous sources well-known to the art (supra). The glycolate oxidase used in the reaction should be present in an effective concentration, usually a concentration of about 0.01 to about 1000 IU/mL, preferably about 0.1 to about 10 IU/mL. An IU (International Unit) is defined as the amount of enzyme that will catalyze the transformation of one micromole of substrate per minute. A procedure for the assay of this enzyme is found in I. Zelitch and S. Ochoa, J. Biol. Chem., Vol. 201, 707-718 (1953). This method is also used to assay the activity of recovered or recycled glycolate oxidase.

Optimal results in the use of glycolate oxidase as a catalyst for the oxidative conversion of L-lactic acid to pyruvic acid are obtained by incorporating into the reaction solution a catalyst for the decomposition of hydrogen peroxide. One such peroxide-destroying catalyst which is effective in combination with glycolate oxidase is the enzyme catalase (E.C. 1.11.1.6). Catalase catalyzes the decomposition of hydrogen peroxide to water and oxygen, and it is believed to improve yields of pyruvic acid in the present process by accelerating the decomposition of the hydrogen peroxide produced along with pyruvic acid in the glycolate oxidase-catalyzed reaction of lactic acid with O₂. The concentration of catalase should be 50 to 50,000 IU/mL, preferably 2,000 to 15,000 IU/mL. It is preferred that the catalase and glycolate oxidase concentrations be adjusted within the above ranges so that the ratio (measured in IU for each enzyme) of catalase to glycolate oxidase is at least about 250:1.

In addition to using soluble enzymes as catalysts, microbial transformants which express glycolate oxidase activity as well as endogenous catalase activity have been prepared, and their use as a microbial catalyst in the present invention demonstrated. A microbial cell catalyst which has been utilized in the present invention is a transformant of *Hansenula polymorpha* (a methylotrophic yeast). Several transformants of *H. polymorpha* having sufficient glycolate oxidase activity have been prepared by inserting the DNA for glycolate oxidase into an expression vector under the control of the formate dehydrogenase (FMD) promoter. *H. polymorpha* was transformed with this vector and a strain producing high levels of glycolate oxidase was selected and designated *H. polymorpha* GO1 (deposited in ARS Patent Culture Collection, under the Northern Regional Research Laboratory accession number: Y-21065, with the U.S.D.A. at Peoria, Illinois).

*H. polymorpha* cell catalysts were typically prepared by first growing an inoculum of an *H. polymorpha* transformant in 500 mL of YPD (Difco), pH 4.4. This culture was then inoculated into a fermenter containing 10 L of Yeast Nitrogen Base (YNB, Difco) without amino acids (14 g), ammonium sulfate (50 g) and methanol (100 g), at pH 5.0. The fermenter was operated for 42.5 hours at 37°C, an agitation rate of 400 rpm, constant pH of 5.0, 40 % dissolved oxygen (controlled), and 2 x 10⁵ Pa (14 psig) of air. At the conclusion of the fermentation, 1.0 kg of glycerol was added and the cells harvested by centrifugation, frozen in liquid nitrogen, and stored at -80 °C.

A second microbial cell catalyst which has been utilized in the present invention is a transformant of *Pichia pastoris* (a methylotrophic yeast) which expresses the glycolate oxidase enzyme from spinach, as well as an endogenous catalase. Several transformants of *P. pastoris* having sufficient glycolate oxidase activity been prepared by inserting a DNA fragment containing the spinach glycolate oxidase gene into a *P*. *pastoris* expression vector (pHIL-D4) such as to be under control of the methanol inducible alcohol oxidase I promoter, generating the plasmid pMP1. *P. pastoris* strain GTS115 (NRRL Y-15851) was transformed by plasmid pMP1 and a selection was done as to allow integration of the linearized plasmid pMP1 into the chromosomal alcohol oxidase I locus and replacement of alcohol oxidase gene with glycolate oxidase gene. A pool of such transformants were next selected for maximal number of integrated copies of the expression cassette. A high copy number transformant designated *P. pastoris* strain GS115-MSP10 was isolated and deposited as NRRL Y-21001.

*P. pastoris* cells were typically prepared by growing an inoculum in 100 mL of YNB containing 1 % glycerol. After 48 hours growth at 30 °C, the cells were transferred into a fermenter containing 10 L of media composed of yeast nitrogen base (YNB) without amino acids (134 g), glycerol (100 g), and biotin (20 mg). The fermentation was operated at pH 5.0 (controlled with NH₄OH), 30 °C, agitation rate of 200 rpm, aeration of 5 slpm, 1.4 x 10⁵ Pa (5 psig) of air, and dissolved oxygen maintained at no lower than 50 % saturation. When glycerol was depleted, the cells were induced to express glycolate oxidase by growth in the same media except that methanol (50 g) was substituted for glycerol. Glycolate oxidase activity during induction was followed by enzyme assay. After 24 hours of induction the cells were harvested following treatment with glycerol (1 kg). Following harvest the cells were frozen in liquid nitrogen and stored at -80 °C.

*H. polymorpha* and *P. pastoris* cell transformants required permeabilization prior to use as catalyst for the oxidation of glycolic acid to glyoxylic acid. A variety of known methods of permeabilization were useful for preparing cells with sufficient glycolate oxidase activity (see Felix, H., Anal. Biochemistry, Vol. 120, 211-234, (1982)). Typically, a suspension of 10 wt % wet cells in 0.1 % (v/v) "TRITON" X - 100/20 mM phosphate buffer (pH 7.0) was mixed for 15 minutes, then frozen in liquid nitrogen, thawed, and washed with 20 mM phosphate/0.1 mM FMN buffer (pH 7.0). A second method of permeabilization was performed by mixing a suspension of 10 wt % wet cells in 0.2 % (w/v) benzalkonium chloride/20 mM phosphate buffer (pH 7.0) for 60 minutes, then washing the permeabilized cells with 20 mM phosphate/0.1 mM FMN buffer (pH 7.0). Once permeabilized, the amount of whole cell catalyst added to a reaction mixture was chosen so as to provide the necessary concentrations of glycolate oxidase and catalase activities as described above for the corresponding soluble enzymes. Recoveries of glycolate oxidase and catalase activities of greater than 100 % of their initial values are due to increased permeabilization of the cells during the course of the reaction.

Microbial cell transformants were assayed for glycolate oxidase activity by accurately weighing ca. 5-10 mg of wet cells (blotted on filter paper to remove excess moisture) into a 3-mL quartz cuvette containing a magnetic stirring bar and 2.0 mL of a solution which was 0.12 mM in 2,6-dichlorophenol-indophenol (DCIP) and 80 mM in TRIS (tris(hydroxymethyl)aminomethane) buffer (pH 8.3). The cuvette was capped with a rubber septum and the solution deoxygenated by bubbling with nitrogen for 5 minutes. To the cuvette was then added by syringe 40 µ L of 1.0 M glycolic acid/1.0 M TRIS (pH 8.3), and the mixture stirred while measuring the change in absorption with time at 605 nm (ε = 22,000). Catalase activity was assayed by accurately weighing ca. 2-5 mg of wet cells (blotted on filter paper to remove excess moisture) into a 3-mL quartz cuvette containing a magnetic stirring bar and 2.0 mL of a distilled water, then adding 1.0 mL of 59 mM hydrogen peroxide in 50 mM phosphate buffer (pH 7.0) and measuring the change in absorption with time at 240 nm (ε = 39.4). Glycolate oxidase and catalase activities of the *H. polymorpha* or *P. pastoris* wet cells (permeabilized) cultured in different media ranged from 20 to 120 DCIP IU/gram wet cells for glycolate oxidase and 30,000 to 200,000 IU/gram wet cells for endogenous catalase.

An optional but often beneficial ingredient in the reaction solution is flavin mononucleotide (FMN), which is generally used at a concentration of up to about 2.0 mM, preferably about 0.01 to about 0.2 mM. It is believed the FMN increases the productivity of the glycolate oxidase, by which is meant the amount of glycolic acid converted to glyoxylic acid per unit of enzyme. It is to be understood that the concentration of added FMN is in addition to any FMN present with the enzyme, because FMN is often also added to the enzyme during the preparation of the enzyme. The structure of FMN and a method for its analysis is found in K. Yagai, Methods of Biochemical Analysis, Vol. X, Interscience Publishers, New York, 1962, p. 319-355.

L-lactic acid is available commercially. In the present reaction its initial concentration is in the range of 0.10 M to 2.0 M, preferably between 0.25 M and 1.0 M. It can be employed in the reaction as the acid, or as a compatible salt thereof; that is, a salt that is water-soluble and whose cation does not interfere with the desired conversion of L-lactic acid to pyruvic acid. Suitable and compatible salt-forming cationic groups are readily determined by trial. Representative of such salts are the alkali metal, alkaline earth metal, ammonium. substituted ammonium, phosphonium, and substituted phosphonium salts. L-lactic acid produced via fermentation can be used as substrate as a filtered solution directly from the fermenter, without purification or isolation from the fermentation broth.

The conversion of L-lactic acid to pyruvic acid is conveniently and preferably conducted in aqueous media. The pH of the reaction mixture is adjusted to a value between 6 and 10, preferably between 7 and 9. Within this pH range, the exact value may be adjusted to obtain the desired pH by adding any compatible, non-interfering base, including (but not limited to) alkali metal hydroxides, carbonates, bicarbonates and phosphates. The pH of an unbuffered reaction mixture decreases by ca. 2 pH units as the reaction proceeds, so it is often useful to start the reaction near the high end of the maximum enzyme activity pH range, about 9.0 - 8.5, and allow it to drop during the reaction; typically, the final pH of unbuffered reaction mixtures ranges from ca. 6.7 to 7.5. The pH can optionally be maintained by the separate addition of a non-interfering inorganic or organic buffer which has some buffering capacity around the pH of 7.5, since the optimal enzyme activity for the oxidation of L-lactate is close to this value; an initial pH of 7.5 is employed when using a suitable buffer. It is understood that L-lactic and pyruvic acid are highly dissociated in water, and that at a pH of between 7 and 10 are largely if not substantially present as L-lactate and pyruvate ions.

Oxygen (O₂), the oxidant for the conversion of L-lactic acid to pyruvic acid, may be added as a gas to the reaction by agitation of the liquid at the gas-liquid interface or through a membrane permeable to oxygen. When employing permeabilized whole-cell catalyst, oxygen may be added by sparging (bubbling) oxygen or an oxygen containing gas through the reaction mixture. Under most conditions, the reaction rate is at least partially controlled by the rate at which oxygen can be dissolved into the aqueous medium. Thus, although oxygen can be added to the reaction as air, a relatively pure form of oxygen may also be used. Although no upper limit of oxygen pressure is known, oxygen pressures up to 50 x 10⁵ Pa (50 atmospheres) may be used, and an upper limit of 15 x 10⁵ Pa (15 atmospheres) is preferred. Agitation is important to maintaining a high oxygen dissolution (hence reaction) rate. Any convenient form of agitation is useful, such as stirring. High shear agitation or agitation that produces foam may decrease the activity of the soluble enzyme(s), and should be avoided when using soluble enzyme catalysts.

The reaction temperature is an important variable, in that it affects reaction rate and the stability of the enzymes. Typically a reaction temperature of up to about 40 °C may be used without substantial loss of catalytic activity, while the preferred reaction temperature range is from about 0 °C to about 15 °C. Operating in the preferred temperature range maximizes recovered enzyme activity at the end of the reaction. The temperature should not be so low that the aqueous solution starts to freeze. Temperature can be controlled by ordinary methods, such as, but not limited to, by using a jacketed reaction vessel and passing liquid of the appropriate temperature through the jacket. The reaction vessel may be constructed of any material that is inert to the reaction ingredients.

Upon completion of the reaction, soluble enzyme catalysts may be removed by filtration or centrifugation and optionally reused. Alternatively, they can be denatured and precipitated by heating, e.g. to 70 °C for 5 minutes and/or can be allowed to remain in the reaction mixture if their presence is not objectionable. Permeabilized cell catalysts may be separated from reaction mixtures for recycle by centrifugation or filtration. Following the removal of the soluble enzyme or microbial cell catalysts, flavin mononucleotide (FMN) may optionally be removed by contacting the solution with activated carbon. The desired pyruvic acid (i.e., the pyruvic acid and pyruvate salts) can then be recovered as the solution, *per se,* or the resulting solution can be concentrated and the pyruvic acid recovered by removal of water; again for example, by stripping of water under reduced pressure, lyophilization (freeze drying) or any other method as generally known in the art.

In the following examples, which serve to further illustrate the invention, the yields of pyruvate and acetate, and the recovered yield of L-lactate, are percentages based on the total amount of L-lactic acid present at the beginning of the reaction, unless otherwise indicated. Analyses of reaction mixtures were performed using high pressure liquid chromatography (HPLC): organic acid analyses were performed using a Bio-Rad HPX-87H column.

### Example 1

Into a 3 oz. Fischer-Porter glass aerosol reaction vessel was placed a magnetic stirring bar and 10 mL of an aqueous solution containing lithium L-lactate (0.75 M), FMN (0.01 mM), isobutyric acid (HPLC internal standard, 0.100 M), bicine buffer (0.788 M), spinach glycolate oxidase (1.0 IU/mL), and *Aspergillus niger* catalase (1,400 IU/mL) at pH 8.9. The reaction vessel was sealed and the reaction mixture was cooled to 5 °C, then the vessel was flushed with oxygen by pressurizing to 5.8 x 10⁵ Pa (70 psig) and venting to atmospheric pressure five times with stirring. The vessel was then pressurized to 5.8 x 10⁵ Pa (70 psig) of oxygen and the mixture stirred at 5 °C. Aliquots (0.10 mL) were removed by syringe through a sampling port (without loss of pressure in the vessel) at regular intervals for analysis by HPLC to monitor the progress of the reaction. After 28.5 hours, the HPLC yields of pyruvate and acetate were 47.7% and 43.6%, respectively, and 11.5% lactate remained. The remaining activity of glycolate oxidase and catalase were 40% and 100%, respectively, of their initial values.

### Example 2

The procedure described in Example 1 was repeated using a 10 mL aqueous solution containing L-lactic acid (96 % L-isomer, 4 % D-isomer, 0.750 M), KH₂PO₄ (0.750 M), FMN (0.01 mM), isobutyric acid (HPLC intemal standard, 0.100 M), spinach glycolate oxidase (1.0 IU/mL), and soluble *Aspergillus niger* catalase (14,000 IU/mL) at pH 8.1 and at 5 °C. After 48 hours, the HPLC yields of pyruvate and acetate were 79.6 % and 3.8 %, respectively, and 20.2 % lactate remained. The remaining activities of glycolate oxidase and catalase after 18 hours of reaction were 22 % and 100 %, respectively, of their initial values.

### Example 3

The procedure described in Example 1 was repeated using a 10 mL aqueous solution containing L-lactic acid (96 % L-isomer, 4 % D-isomer, 0.500 M), KH₂PO₄ (0.50 M), FMN (0.01 mM), isobutyric acid (HPLC internal standard, 0.100 M), spinach glycolate oxidase (2.0 IU/mL), and soluble *Aspergillus niger* catalase (14,000 IU/mL) at pH 8.3 and at 5 °C. After 18 hours, the HPLC yields of pyruvate and acetate were 90.5 % and 4.2 %, respectively, and 6.4 % lactate remained. The remaining activities of glycolate oxidase and catalase were 57 % and 100 %, respectively, of their initial values.

### Example 4

The procedure described in Example 1 was repeated using a 10 mL aqueous solution containing sodium L-lactate (0.500 M), isobutyric acid (HPLC internal standard, 0.100 M), spinach glycolate oxidase (2.0 IU/mL), and soluble *Aspergillus niger* catalase (20,000 IU/mL) at pH 9.0 (adjusted with 50 % NaOH) and at 15 °C; no buffer was added. After 7 hours, the HPLC yields of pyruvate and acetate were 91.6 % and 0.6 %, respectively, and 7.1 % lactate remained. The remaining activities of glycolate oxidase and catalase were 21 % and 100 %, respectively, of their initial values.

### Example 5

The procedure described in Example 1 was repeated using a 10 mL aqueous solution containing sodium L-lactate (0.500 M), isobutyric acid (HPLC internal standard, 0.100 M), spinach glycolate oxidase (6.0 IU/mL), and soluble *Aspergillus niger* catalase (10,000 IU/mL) at pH 9.0 (adjusted with 50 % NaOH) and at 15 °C; no buffer was added. After 5 hours, the HPLC yields of pyruvate and acetate were 95.3 % and 0.9 %, respectively, and 4.5 % lactate remained. The remaining activities of glycolate oxidase and catalase were 68 % and 100 %, respectively, of their initial values.

### Example 6

Into a 3 oz. Fischer-Porter glass aerosol reaction vessel was placed a magnetic stirring bar and 10 mL of an aqueous solution containing sodium L-lactate (0.500 M), KH₂PO₄ (0.50 M), and isobutyric acid (HPLC internal standard, 0.100 M) at pH 9.0 (adjusted with 50% NaOH), and the solution cooled to 5 °C. To the vessel was then added 0.75 g of *Pichia pastoris* transformant GS115-MSP10 (65.2 IU glycolate oxidase and 101,000 IU catalase) which had been permeabilized by treatment with 0.1 % benzalkonium chloride ("LONZA BARQUAT" OJ-50), then the reaction vessel was sealed and the reaction mixture was cooled to 5 °C. The vessel was flushed with oxygen by pressurizing to 5.8 x 10⁵ Pa (70 psig) and venting to atmospheric pressure five times with stirring, then the vessel was pressurized to 5.8 x 10⁵ Pa (70 psig) of oxygen and the mixture stirred at 5 °C. Aliquots (0.10 mL) were removed by syringe through a sampling port (without loss of pressure in the vessel) at regular intervals for analysis by HPLC to monitor the progress of the reaction. After 5 hours, the HPLC yields of pyruvate and acetate were 97.6 % and 2.5 %, respectively, and 0.3 % lactate remained. The remaining permeabilized cell activity of glycolate oxidase and catalase were 104 % and 105 %, respectively, of their initial values.

### Example 7

The reaction in Example 6 was repeated using bicine buffer (0.5 M) in place of KH₂PO₄ (0.50 M). After 5 hours, the HPLC yields of pyruvate and acetate were 93.1 % and 6.3 %, respectively, and 0.4 % lactate remained. The remaining permeabilized cell activity of glycolate oxidase and catalase were 107 % and 122 %, respectively, of their initial values.

### Example 8

The procedure described in Example 6 was repeated using 10 mL of an aqueous solution containing sodium L-lactate (0.500 M) and isobutyric acid (HPLC internal standard, 0.100 M) at pH 9.0 (adjusted with 50 % NaOH), to which was added 0.75 g of *Pichia pastoris* transformant GS115-MSP10 (65.2 IU glycolate oxidase and 101,000 IU catalase) which had been permeabilized by treatment with 0.1 % benzalkonium chloride ("LONZA BARQUAT" OJ-50); no buffer was added. After 5 hours, the HPLC yields of pyruvate and acetate were 99.0 % and 0.7 %, respectively, and 0.4 % lactate remained. The remaining penneabilized cell activity of glycolate oxidase and catalase were 119 % and 113 %, respectively, of their initial values.

### Example 9

The procedure described in Example 6 was repeated using 10 mL of an aqueous solution containing sodium L-lactate (0.500 M) and isobutyric acid (HPLC internal standard, 0.100 M) at pH 9.0 (adjusted with 50 % NaOH), to which was added 0.35 g of *Pichia pastoris* transformant GS115-MSP10 (22.6 IU glycolate oxidase and 50,000 IU catalase) which had been permeabilized by treatment with 0.1 % benzalkonium chloride ("LONZA BARQUAT" OJ-50); no buffer was added. After 8 hours, the HPLC yields of pyruvate and acetate were 97.4 % and 2.3 %, respectively, and 0.4 % lactate remained. The remaining permeabilized cell activity of glycolate oxidase and catalase were 123 % and 150 %, respectively, of their initial values.

### Example 10

The procedure described in Example 6 was repeated using 10 mL of an aqueous solution containing sodium L-lactate (0.500 M) and isobutyric acid (HPLC internal standard, 0.100 M) at pH 9.0 (adjusted with 50 % NaOH), to which was added 0.18 g of *Pichia pastoris* transformant GS115-MSP10 (11.3 IU glycolate oxidase and 25,000 IU catalase) which had been permeabilized by treatment with 0.1 % benzalkonium chloride ("LONZA BARQUAT" OJ-50); no buffer was added. After 10 hours, the HPLC yields of pyruvate and acetate were 92.9 % and 5.0 %, respectively, and 3.3 % lactate remained. The remaining permeabilized cell activity of glycolate oxidase and catalase were 121 % and 228 %, respectively, of their initial values.

### Example 11

The procedure described in Example 6 was repeated using 10 mL of an aqueous solution containing sodium L-lactate (1.00 M) and isobutyric acid (HPLC internal standard, 0.100 M) at pH 9.0 (adjusted with 50 % NaOH), to which was added 0.71 g of *Pichia pastoris* transformant GS115-MSP10 (45.9 IU glycolate oxidase and 100,000 IU catalase) which had been permeabilized by treatment with 0.1 % benzalkonium chloride ("LONZA BARQUAT" OJ-50); no buffer was added. After 8 hours, the HPLC yields of pyruvate and acetate were 89.1 % and 8.4 %, respectively, and 1.3 % lactate remained. The remaining permeabilized cell activity of glycolate oxidase and catalase were 124 % and 145 %, respectively, of their initial values.

### Example 12

The procedure described in Example 6 was repeated using 10 mL of an aqueous solution containing sodium L-lactate (0.50 M) and isobutyric acid (HPLC internal standard, 0.100 M) at pH 9.0 (adjusted with 50 % NaOH), to which was added 0.66 g of *Pichia pastoris* transformant GS115-MSP10 (62.7 IU glycolate oxidase and 100,000 IU catalase) which had been permeabilized by treatment with 0.1 % benzalkonium chloride ("LONZA BARQUAT" OJ-50); no buffer was added. The reaction temperature was 15 °C and the oxygen pressure was 5.8 x 10⁵ Pa (70 psig). After 3 hours, the HPLC yields of pyruvate and acetate were 98.2 % and 1.2 %, respectively, and 0.6 % lactate remained. The remaining permeabilized cell activity of glycolate oxidase and catalase were 124 % and 130 %, respectively, of their initial values.

### Example 13

The procedure described in Example 12 was repeated using 10 mL of an aqueous solution containing sodium L-lactate (0.50 M) and isobutyric acid (HPLC internal standard, 0.100 M) at pH 9.0 (adjusted with 50 % NaOH), to which was added 1.04 g of *Hansenula polymorpha* transformant GO1 (64.7 IU glycolate oxidase and 50,000 IU catalase) which had been permeabilized by treatment with 0.1 % benzalkonium chloride ("LONZA BARQUAT" OJ-50); no buffer was added. The reaction temperature was 15 °C and the oxygen pressure was 5.8 x 10⁵ Pa (70 psig). After 2 hours, the HPLC yields of pyruvate and acetate were 97.0 % and 2.5 %, respectively, and 0.4 % lactate remained. The remaining permeabilized cell activity of glycolate oxidase and catalase were 99 % and 155 %, respectively, of their initial values.

### Example 14

The reaction described in Example 13 was repeated at 5 °C and 9.3 x 10⁵ Pa (120 psig) of oxygen. After 4 hours, the HPLC yields of pyruvate and acetate were 93.1 % and 3.7 %, respectively, and 2.2 % lactate remained. The remaining permeabilized cell activity of glycolate oxidase and catalase were 66 % and 180 %, respectively, of their initial values.

### Example 15

The reaction described in Example 13 was repeated at 30 °C and 5.8 x 10⁵ Pa (70 psig) of oxygen. After 3 hours, the HPLC yields of pyruvate and acetate were 89.9 % and 6.5 %, respectively, and 0.6 % lactate remained. The remaining permeabilized cell activity of glycolate oxidase and catalase were 45 % and 140 %, respectively, of their initial values.

### Example 16

A 300-mL EZE-Seal stirred autoclave reactor equipped with Dispersimax Impeller (Autoclave Engineers) was charged with 100 mL of a solution containing sodium L-lactate (5.50 g, 0.50 M). To the reactor was then added 6.70 g of *Pichia pastoris* transformant strain GS115-MSP10 (670 IU glycolate oxidase and 1,177,000 IU catalase) which had been permeabilized by treatment with 0.1 % benzalkonium chloride ("LONZA BARQUAT" MB-50), and the mixture adjusted to pH 9.0 with 50 % NaOH and cooled to 5°C. The reactor purged with oxygen, then the mixture was stirred at 750 rpm, which bubbled oxygen through the mixture via the action of the turbine impeller, and at 5 °C under 3.8 x 10⁵ Pa (40 psig) of oxygen. The reaction was monitored by taking a 0.40 mL aliquot of the reaction mixture at regular intervals, filtering the aliquot using a Millipore Ultrafree-MC 10,000 NMWL Filter Unit, and analyzing the filtrate by HPLC using 0.10 M isobutyric acid added to the sample as internal standard. After 3.0 hours, the HPLC yields of pyruvate and acetate were 99.2 % and 1.4 %, respectively, and 0.6 % lactate remained. The recovered activities of permeabilized-cell glycolate oxidase and catalase were 107 % and 106 % of their initial values, respectively.

The reaction mixture was centrifuged to remove the permeabilized-cell catalyst, and the resulting supernatant filtered through a 0.2 mm- nylon filter. The pH of the resulting filtrate was adjusted to 4.6 with 1.0 N HCl, then the solution was frozen and the water removed by lyophilization to produce 5.20 g of sodium pyruvate (96 % isolated yield, 98 % sodium pyruvate as determined by HPLC analysis).

### Example 17

A fermentation broth containing 109.9 g/L of ammonium lactate (97.8 % L-lactate, 2.2 % D-lactate), 0.8 g/L acetate, and 2.8 g/L maltose was centrifuged to remove particulate matter, then filtered through a 0.45 mm filter. The concentration of ammonium lactate in the resulting solution was 1.10 M (118-6 g/L determined by HPLC analysis). Into a 300-mL EZE-Seal stirred autoclave reactor equipped with Dispersimax Impeller (Autoclave Engineers) was placed 45 mL of the 1.10 M filtered fermentation broth, then 55 mL of distilled water was added to produce 100 mL of an aqueous solution containing 0.50 M ammonium lactate. To the reactor was then added 6.70 g of recycled *Pichia pastoris* transformant strain GS115-MSP10 (666 IU glycolate oxidase and 1,107,000 IU catalase) which had been permeabilized by treatment with 0.1 % benzalkonium chloride ("LONZA BARQUAT" OJ-50), and the mixture adjusted to pH 7.5 with 50% NaOH and cooled to 5°C. The reactor purged with oxygen, then the mixture was stirred at 750 rpm, which bubbled oxygen through the mixture via the action of the turbine impeller, and at 5°C under 3.8 x 10⁵ Pa (40 psig) of oxygen. The reaction was monitored by taking a 0.40 mL aliquot of the reaction mixture at regular intervals, filtering tlie aliquot using a Millipore Ultrafree-MC 10,000 NMWL Filter Unit, and analyzing the filtrate by HPLC using 0.10 M isobutyric acid as internal standard. After 3.0 hours, the HPLC yields of pyruvate and acetate were 94.1 % (96.2 % based on L-lactate) and 2.8 %, respectively, and 2.5 %, lactate remained. The recovered activities of permeabilized-cell glycolate oxidase and catalase were 101 % and 56 % of their initial values, respectively.

## Claims

1. A process for the production of pyruvic acid comprising the steps of reacting, in an aqueous solution, L-lactic acid and oxygen in the presence of a permeabilized whole cell catalyst comprising an enzyme catalyst glycolate oxidase and an enzyme catalyst catalase, with or without a buffer, at a temperature from about 0°C to about 40°C, for a time sufficient to convert the L-lactic acid to pyruvic acid at high yields, wherein the initial concentration of the L-lactic acid is from about 0.1 to about 2.0 M, and then recovering the pyruvic acid.

2. A process of Claim 1 wherein said enzyme catalysts are present in a microbial transformant.

3. A process of Claim 1 wherein no additional buffer is added and no pH control occurs during the reaction.

4. A process of Claim 1 wherein the reaction is performed at a pH of about 6 to about 10 and wherein from 0.01 to 1,000 IU/ml of glycolate oxidase activity and from 50 to 50,000 IU/ml of catalase activity are present.

5. A process of Claim 3 wherein from 0.1 to 10 IU/ml of glycolate oxidase activity and from 2,000 to 15,000 IU/ml of catalase activity are present and the IU/ml ratio of catalase to glycolate oxidase activities is at least 250:1.

## Patentansprüche

1. Verfahren zur Herstellung von Brenztraubensäure, umfassend die Schritte des Umsetzens von L-Milchsäure und Sauerstoff in einem wässrigen Medium in Gegenwart eines permeabiliserten Ganzzellenkatalysators, der einen Enzymkatalysator Glykolat-Oxidase und einen Enzymkatalysator Katalase mit oder ohne Puffer aufweist, und zwar bei einer Temperatur von etwa 0°C bis etwa 40°C für eine ausreichende Zeitdauer, um die L-Milchsäure in Brenztraubensäure mit hohen Ausbeuten umzuwandeln, wobei die Ausgangskonzentration der L-Milchsäure etwa 0,1 bis etwa 2,0 M beträgt; sowie nachfolgendes Gewinnen der Brenztraubensäure.

2. Verfahren nach Anspruch 1, bei welchem die Enzymkatalysatoren in einem mikrobiellen Transformanten vorliegen.

3. Verfahren nach Anspruch 1, bei welchem während der Reaktion kein zusätzlicher Puffer zugesetzt wird und keine pH-Wertkontrolle erfolgt.

4. Verfahren nach Anspruch 1, bei welchem die Reaktion ausgeführt wird bei einem pH-Wert von etwa 6 bis etwa 10 und bei welchem etwa 0,01 bis 1.000 IU/ml Glykolat-Oxidaseaktivität und etwa 50 bis 50.000 IU/ml Katalaseaktivität vorliegen.

5. Verfahren nach Anspruch 3, bei welchem 0,1 bis 10 IU/ml Glykolat-Oxidaseaktivität und 2.000 bis 15.000 IU/ml Katalaseaktivität vorliegen und bei welchem das IU/ml Verhältnis der Katalase- zu Glykolat-Oxidaseaktivitäten mindestens 250:1 beträgt.

## Revendications

1. Un procédé de préparation d'acide pyruvique comprenant les étapes suivantes: réaction, dans une solution aqueuse, de l'acide L-lactique et de l'oxygène en présence d'un catalyseur à base de cellules intégrales perméabilisées comprenant un catalyseur enzymatique glycolate oxydase et un catalyseur enzymatique de catalase, en présence ou en absence d'un tampon et à une température d'environ 0°C à environ 40°C, pendant un temps suffisant pour convertir l'acide L-lactique en acide pyruvique selon des rendements élevés, dans lequel la concentration initiale de l'acide L-lactique est comprise entre environ 0,1 et environ 2,0 M et ensuite récupération de l'acide pyruvique.

2. Un procédé selon la revendication 1, dans lequel lesdits catalyseurs enzymatiques sont présents sous forme d'un transformant microbien.

3. Un procédé selon la revendication 1, dans lequel on n'ajoute aucun tampon supplémentaire et on n'exerce aucune régulation du pH pendant la réaction.

4. Un procédé selon la revendication 1, dans lequel la réaction est mise en oeuvre à un pH d'environ 6 à environ 10 et dans lequel sont présents de 0,01 à 1 000 IU/mil d'activité de glycolate oxydase et de 50 à 50 000 IU/ml d'activité de catalase.

5. Un procédé selon la revendication 3, dans lequel sont présentes de 0,1 à 10 IU/ml d'activité de glycolate oxydase et de 2 000 à 15 000 IU/ml d'activité de catalase et le rapport de l'activité de la catalase à l'activité de la glycolate oxydase en IU/ml est d'au moins 250/l.
